# EUROPÄISCHE PATENTSCHRIFT

(11) **EP 0 574 852 B1**
(45) Veröffentlichungstag und Bekanntmachung des Hinweises auf die Patenterteilung: **15.03.2000**
(21) Anmeldenummer: 93109451.0
(22) Anmeldetag: 14.06.1993
(51) Int. Cl.: C12Q 1/68

(54) **Spezifischer Nachweis von Neisseria gonorrhoeae**
Specific detection of Neisseria gonorrhoeae
Détection spécifique de Neisseria gonorrhoeae

(30) Priorität: 17.06.1992 DE 4219821
(43) Veröffentlichungstag der Anmeldung: 22.12.1993
(73) Patentinhaber: Roche Diagnostics GmbH, 68298 Mannheim (DE)
(72) Erfinder: Stern, Anne, Dr., D-8122 Penzberg (DE); Wolff, Karin, D-8034 Germering (DE)

(56) Entgegenhaltungen:
- EP-A- 0 337 896
- EP-A- 0 408 077
- EP-A- 0 452 596
- WO-A-90/14442
- JOURNAL OF CLINICAL MICROBIOLOGY, Bd.29, Nr.5, Mai 1991, WASHINGTON, DC Seiten 883 - 888 PANKE ET AL. 'Comparison of gen-probe DNA probe test and ....'
- JOURNAL OF GENERAL MICROBIOLOGY, Bd.135, Nr.6, Juni 1989, LONDON Seiten 1735 - 1745 ROSSAU ET AL. 'Specific Neisseria gonorrhoeae DNA-probes derived from ribosomal RNA'

SEQUENZPROTOKOLL

(1) ALLGEMEINE INFORMATION:

(i) ANMELDER:
(A) NAME: BOEHRINGER MANNHEIM GMBH
(B) STRASSE: Sandhofer Str. 116
(C) ORT: Mannheim
(D) BUNDESLAND: Deutschland
(F) POSTLEITZAHL: D-6800

(ii) ANMELDETITEL: Spezifischer Nachweis von Neisseria
Gonorrhoeae

(iii) ANZAHL DER SEQUENZEN: 15

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(vi) FRÜHERE ANMELDEDATEN:
(A) ANMELDENUMMER: DE P4219821.6
(B) ANMELDEDATUM: 17-JUN-1992

(2) INFORMATION ZU SEQ ID NO: 1:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 1:

GCTGTGGGTA GGGGTGA
17

(2) INFORMATION ZU SEQ ID NO: 2:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 2:

TCACCCCTAC CCACAGC
17

(2) INFORMATION ZU SEQ ID NO: 3:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 3:

CAGGTGGGTA GGATGAG
17

(2) INFORMATION ZU SEQ ID NO: 4:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 4:

CTCATCCTAC CCACCTG
17

(2) INFORMATION ZU SEQ ID NO: 5:

(i) SEQUENZ CHARAKTERISTIKA:
(A) LÄNGE: 16 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 5:

GTAGGCTGAT GAAGGT
16

(2) INFORMATION ZU SEQ ID NO: 6:

(i) SEQUENZ CHARAKTERISTIKA:
(A) LÄNGE: 16 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 6:

ACCTTCATCA GCCTAC
16

(2) INFORMATION ZU SEQ ID NO: 7:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 7:

ATCCGGGTTT TCTTAACA
18

(2) INFORMATION ZU SEQ ID NO: 8:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 8:

TGTTAAGAAA ACCCGGAT
18

(2) INFORMATION ZU SEQ ID NO: 9:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 9:

ACAAGTCGGG CAGGTGC
17

(2) INFORMATION ZU SEQ ID NO: 10:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 10:

GCACCTGCCC GACTTGT
17

(2) INFORMATION ZU SEQ ID NO: 11:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 11:

GAAGGACTTC AAGAGAT
17

(2) INFORMATION ZU SEQ ID NO: 12:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 12:

ATCTCTTGAA GTCCTTC
17

(2) INFORMATION ZU SEQ ID NO: 13:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 13:

CGATTTGCAA CAGTTTA
17

(2) INFORMATION ZU SEQ ID NO: 14:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 14:

TAAACTGTTG CAAATCG
17

(2) INFORMATION ZU SEQ ID NO: 15:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS zu rRNS

(xi) SEQUENZBESCHREIBUNG: SEQ ID NO: 15:

TTCTCGGTGT TAAGAAA
17

## Beschreibung

Die Erfindung betrifft spezifische Nukleinsäure-Sonden zum Nachweis des Erregers Neisseria gonorrhoeae sowie ein Verfahren zum Nachweis von Neisseria gonorrhoeae unter Verwendung dieser Sonden.

N.gonorrhoeae ist der Erreger der Gonorrhoe, einer Geschlechtskrankheit, die zu den häufigsten meldepflichtigen Infektionskrankheiten der Welt zählt. Allein die Zahl der Neuinfektionen liegt bei 60 - 65 Millionen pro Jahr (World Health Statisticals Annual, 1979; Herrmann, Innere Medizin in Praxis und Klinik; Hornbostel H., Kaufmann W., Siegenthaler W., eds., Thieme Verlag, 1985, 59-62). Die Symptome sind abhängig von dem Geschlecht des Infektonsträgers. Während beim Mann sich die genitale Infektion vor allem durch eine eitrige Entzündung und Schwellung der Urethramündung bemerkbar macht, treten bei der Frau vor allem Entzündungen der Zervix, z.T. aber auch der Urethra auf. Bei der Frau treten in 50% der Infektionsfälle keine oder nur geringfügige Symptome auf. Bei 10 bis 15% der Frauen greift die Infektion auf die Eileiter über, wodurch die Gefahr einer Sterilität besteht. In 1 bis 3% der Fälle tritt bei beiden Geschlechtern eine systemische Invasion durch den Erreger auf, was Arthritis, Endocarditis und Peritonitis zur Folge haben kann. Die Infektionen können häufig auch asymptomatisch verlaufen, wodurch viele Infektionsträger zur Verbreitung der Krankheit beitragen, ohne selbst erkennbar zu erkranken (Davis et al., In: Microbiology, Harper International Edition 1981).

Die Gattung Neisseria stellt eine Gruppe eng verwandter, gramnegativer Diplokokken dar, zu denen sowohl pathogene wie auch apathogene Spezies gehören. Apathogene Neisserien wie z.B. Neisseria flava und elongata besiedeln die Schleimhäute der Mundhöhle, der Atemwege, sowie des Genitaltraktes und zählen zur mikrobiellen Normalflora des Menschen (Kayser, in: Medizinische Mikrobiologie, Wiesmann E., ed., Thieme Verlag, 1986, 104-144). Die enge Verwandtschaft der Neisserien wurde u.a. durch DNA-DNA-Hybridisierungsversuche nachgewiesen (Hoke und Vedros, Int.J.System.Bacteriol. 32 (1982), 57-66). Voraussetzung für eine gezielte Therapie, sowie für die Eindämmung der Infektionsausbreitung ist eine schnelle und zuverlässige Nachweismethode des Erregers N.gonorrhoeae, mit deren Hilfe man effizient und spezifisch N.gonorrhoeae nachweisen und eindeutig von anderen Spezies, vor allem von den anderen Mitgliedern der Gattung Neisseria, unterscheiden kann.

Die bisher verwendeten Nachweistests setzen das Anlegen einer Kultur voraus. Dies bedingt Probleme beim Material-Transport und bei der Anzucht, da die Gonokokken aufgrund ihrer autolytischen Enzymsysteme gegenüber Umwelteinflüssen wie Temperaturveränderung und Austrocknung außerordentlich empfindlich sind. Die Kultivierung von N.gonorrhoeae erfordert ein Selektivmedium (z.B. Thayer-Martin Medium), auf dem jedoch auch apathogene Neisseria-Spezies, wie z.B. N.lactamica wachsen können. Der definitive diagnostische Nachweis erfolgt daher durch Differenzierung mittels Kohlenhydratnutzung, Antigennachweis, Oxidasereaktion oder Fluoreszenz-Antikörper-Screening. All diese Nachweissysteme sowie die erforderliche Kultivierung der zu testenden Mikroorganismen sind zeitaufwendig und müssen weitgehend in mikrobiologischen Fachlaboratorien durchgeführt werden.

Bereits vor einigen Jahren wurde die Möglichkeit erkannt, daß mit Hilfe von Nukleinsäure-Sonden spezifisch Organismen nachgewiesen werden können. Besonders geeignet erscheinen Nukleinsäure-Sonden, welche komplementär zur ribosomalen RNA (rRNA) sind. Diese Sonden weisen den Vorteil einer hohen Sensitivität auf, da in jeder Zelle 1000 bis 10.000 Kopien dieser rRNA, sowie auch mehrere Kopien der für diese rRNA codierenden Gene (rDNA) vorhanden sind.

Jede Zelle besitzt multiple rRNA Operons, die in Eubakterien üblicherweise wie folgt aufgebaut sind: am 5'Ende des Operons ist das 16S rRNA Gen lokalisiert, an das sich das 23S rRNA Gen anschließt; am 3'Ende des Operons liegt das 5S rRNA Gen. Die einzelnen Gene sind durch Spacer-Regionen voneinander getrennt, in denen z.T. transfer RNA (tRNA) Gene und Signalsequenzenzen für das post-transkriptionelle Processing gefunden werden. Das rRNA Operon wird zunächst in eine einzige Precursor-RNA umgeschrieben. Das primäre Transkript wird dann anschließend durch Endo- und Exoribonukleasen in die reifen Produkte prozessiert. Das bedeutet, daß die 23S rRNA Sequenzen und die Spacer-Regionen sowohl als DNA im Genom als auch als RNA-Transkripte vorliegen.

Die Methode zur Identifizierung von Organismen mit Hilfe rRNA-spezifischer Proben wurde bereits mehrfach beschrieben (EP-B 0 155 359, WO 84/02721, EP-A 0 076 123).

Auch zum Nachweis von Neisseria gonorrhoeae wurden Sonden, deren Sequenzen komplementär zu Teilbereichen der rRNA sind, eingesetzt (EP-A 0 272 009). Mit den in EP-A 0 272 009 beschriebenen Sonden konnten jedoch keine eindeutigen Test-Ergebnisse erzielt werden. Zudem ist die Sensitivität des Nachweises unter Verwendung dieser Sonden derjenigen der Kultivierungsmethode unterlegen (Panke et al., J.Clin.Microbiol. 29, (1991), 883-888).

Für Neisseria gonorrhoeae spezifische Oligonukleotid-Sonden sind in der EP-A 0 408 077 beschrieben. Diese Sonden entsprechen Teilbereichen der 16S rRNA von Neisseria gonorrhoeae. Nukleinsäure-Sonden, die mit der 23S rRNA von Neisseria gonorrhoeae hybridisieren, wurden in der EP-A 0 408 077 nicht beschrieben. Derartige Sonden sind jedoch von Interesse, da sie aufgrund der Länge der Sequenzen eine größere Auswahl an spezifischen Sonden erlauben.

In der WO 90/14442 werden Nukleinsäure-Sonden beschrieben, welche Sequenzen der 16S rRNA, sowie aus dem 5' Bereich des 23S rRNA Gens entsprechen. Um eine Differenzierung der Neisseria-Spezies zu erreichen, müssen jedoch extrem stringente Hybridisierungsbedingungen angewendet werden (Hybridisierung in Gegenwart von 0,9 mol/l NaCl bei 60°C, Waschen in Gegenwart von 0,03 mol/l NaCl bei 60°C). Einige der in der WO 90/14442 beschriebenen Sonden hybridisieren selbst unter diesen hochstringenten Bedingungen auch mit der nicht-pathogenen Spezies N.flava. Auch die in der EP-A 0 337 896 beschriebenen 23S rRNA-spezifischen Nukleinsäure-Sonden erfordern hochstringente Hybridisierungsbedingungen und hybridisieren selbst unter diesen Bedingungen nicht spezifisch mit N.gonorrhoeae, sondern auch mit anderen Neisseria-Spezies wie z.B. N.meningitidis. In der EP-A 0 452 596 wird eine Nukleinsäure-Sonde aus der Spacer-Region zwischen dem 16S und 23S rRNA Gen für den Nachweis von N.gonorrhoeae vorgeschlagen, die jedoch auch mit der Nukleinsäure von einigen Stämmen der apathogenen Spezies Neisseria cinerea hybridisiert. Diese bisher beschriebenen 23S-rRNA bzw. rRNA-Spacer-spezifischen Sonden sind daher für einen spezifischen Nachweis von N.gonorrhoeae ungeeignet.

Aufgabe der vorliegenden Erfindung war es daher, Nukleinsäure-Sonden für Neisseria gonorrhoeae bereitzustellen, welche unter den üblichen Hybridisierungsbedingungen eine hohe Spezifität und Sensitivität besitzen und somit einen sicheren qualitativen und quantitativen Nachweis dieses Erregers ermöglichen.

Diese Aufgabe wird gelöst durch eine für Neisseria gonorrhoeae spezifische Nukleinsäure-Sonde, welche mindestens eine der in SEQ ID NO 1-15 gezeigten Sequenzen aufweist. Die Sequenz der Oligonukleotide der Untergruppe SEQ ID NO 1, 3, 5, 7, 9, 11, 13 entsprechen Teilbereichen des 23S rRNA-Gens oder der 3' davon gelegenen Spacer-Region. Die Sequenzen der Oligonukleotide der Untergruppe SEQ ID NO 2, 4, 6, 8, 10, 12, 14 und 15 sind komplementär zu diesen Teilbereichen. Die zelluläre rRNA bzw. deren Precursor lassen sich in einem direkten Hybridisierungsansatz nur mit der letztgenannten Untergruppe nachweisen, während der Nachweis der genomischen DNA, welcher für die entsprechende RNA kodiert, mit beiden Untergruppen möglich ist.

Die Sequenzen der genannten Oligonukleotide sind in Tabelle I wiedergegeben:

**Tabelle I**

| Bezeichnung | Nukleotidsequenz | rDNA-Bereich* |
|---|---|---|
| SEQ ID NO 1 | GCTGTGGGTAGGGGTGA | R |
| SEQ ID NO 2 | TCACCCCTACCCACAGC | R |
| SEQ ID NO 3 | CAGGTGGGTAGGATGAG | R |
| SEQ ID NO 4 | CTCATCCTACCCACCTG | R |
| SEQ ID NO 5 | GTAGGCTGATGAAGGT | R |
| SEQ ID NO 6 | ACCTTCATCAGCCTAC | R |
| SEQ ID NO 7 | ATCCGGGTTTTCTTAACA | R |
| SEQ ID NO 8 | TGTTAAGAAAACCCGGAT | R |
| SEQ ID NO 9 | ACAAGTCGGGCAGGTGC | R |
| SEQ ID NO 10 | GCACCTGCCCGACTTGT | R |
| SEQ ID NO 11 | GAAGGACTTCAAGAGAT | S |
| SEQ ID NO 12 | ATCTCTTGAAGTCCTTC | S |
| SEQ ID NO 13 | CGATTTGCAACAGTTTA | S |
| SEQ ID NO 14 | TAAACTGTTGCAAATCG | S |
| SEQ ID NO 15 | TTCTCGGTGTTAAGAAA | R |

| | | |
|---|---|---|
| * R: Oligonukleotid entspricht der 23S rRNA-Sequenz S: Oligonukleotid entspricht der Spacer-Region | | |

Überraschenderweise gelingt es mit den erfindungsgemäßen Sonden, spezifisch Neisseria gonorrhoeae nachzuweisen. Auch bei den üblichen wenig stringenten Hybridisierungsbedingungen (Hybridisierung in Gegenwart von 0,9 mol/l NaCl bei 40°C, Waschen in Gegenwart von 0,36 mol/l NaCl bei 42-60°C) zeigen diese Sonden keine Hybridisierung mit nahe verwandten Neisseria-Spezies wie z.B. N.flava und N.cinerea. Die sich daraus ergebende hohe Spezifität ist deshalb von großer Bedeutung, weil in der zu untersuchenden Probe neben dem Erreger N.gonorrhoeae auch viele apathogene Neisseria-Spezies vorkommen können, welche Bestandteil der menschlichen Schleimhaut sind.

Die erfindungsgemäßen Sonden sind mindestens 14 Nukleotide lang. Sie können jedoch an ihrem 5'- und/oder 3'-Ende weitere Nukleotide, vorzugsweise bis zu 8 Nukleotide, aufweisen. Eine Gesamtlänge von 30 Nukleotiden sollte aber nicht überschritten werden.

Ein weiterer Gegenstand der Erfindung ist daher eine Neisseria gonorrhoeae-spezifische Nukleinsäure-Sonde, welche mindestens eine der in SEQ ID NO 1-15 gezeigten Sequenzen, sowie an ihrem 5'- und/oder 3'-Ende weitere Nukleotide, vorzugsweise bis zu 8 weitere Nukleotide, aufweist.

Die zusätzlichen Nukleotide der Sonde können hierbei beliebige Nukleotide sein, bevorzugt sind jedoch diejenigen Nukleotide, die den sich auf der rDNA am 5'- bzw. am 3'-Ende befindlichen Nukleotiden bzw. deren komplementären Nukleotiden entsprechen.

Die erfindungsgemäßen, für N.gonorrhoeae spezifischen Nukleinsäure-Sonden können in verschiedenen Formen vorliegen: als einzelsträngige Oligonukleotide oder als doppelsträngige Oligonukleotidfragmente, assoziiert mit anderen Sequenzen, die keine Homologie zur DNA oder RNA aus Neisseria gonorrhoeae aufweisen, wie z.B. Klonierungsvektoren. Bei Verwendung von doppelsträngigen Sonden ist vor der Durchführung der tatsächlichen Nachweisreaktion eine Denaturierung der Sonde unerläßlich. Die erfindungsgemäßen Nukleinsäure-Sonden können sowohl modifizierte als auch nicht modifizierte Ribonukleotide und/oder Desoxyribonukleotide enthalten. Zur Klonierung dieser Nukleinsäuresonden können sowohl Einzelstrang-Vektoren wie z.B. M13 als auch Doppelstrang-Vektoren wie z.B. pBR322 und seine Derivate benutzt werden. Weiterhin können die Sequenzen der in Tabelle 1 aufgeführten Sonden miteinander gekoppelt werden, so daß zwei oder mehr Sonden z.B. in einem Vektor vorliegen.

Ein weiterer Gegenstand der Erfindung ist eine Neisseria gonorrhoeae-spezifische Nukleinsäure-Sonde, welche dadurch gekennzeichnet ist, daß sie eine oder mehrere Kopien der in SEQ ID NO 1-15 gezeigten Sequenzen in einem einzel- oder doppelsträngigen Vektor kloniert enthält.

In einer bevorzugten Ausführungsform der Erfindung sind die Nukleinsäure-Sonden markiert. Hierzu eignen sich alle dem Fachmann bekannten Arten der Markierung, wie der Einbau von radioaktiven Isotopen, oder der Einbau einer nicht-radioaktiven Markierung, wie z.B. eines Digoxigenin-gekoppelten Nukleotids, welches über einen Enzym- oder Fluoreszenz-markierten Antikörper nachgewiesen werden kann.

Ein weiterer Gegenstand der Erfindung ist ein Verfahren zum Nachweis von N.gonorrhoeae durch Hybridisierung von DNA oder RNA der zu untersuchenden Probe mit mindestens einer erfindungsgemäßen Sonde unter üblichen Hybridisierungsbedingungen und Nachweis der Hybridbildung.

Die erfindungsgemäßen Nukleinsäure-Sonden hybridisieren sowohl mit der rRNA als auch mit den entsprechenden rRNA-Genen auf dem bakteriellen Genom. Es ist daher im erfindungsgemäßen Verfahren möglich, über beide Nukleinsäure-Typen die An- und Abwesenheit von N.gonorrhoeae quantitativ und qualitativ zu bestimmen.

Der Nachweis über eine Hybridisierung mit mindestens einer der erfindungsgemäßen Sonden erfolgt nach den üblichen Methoden zum Nachweis von Nukleinsäuren über eine Hybridisierung (Southern, J.Mol.Biol. 98, 1975, 503). Alle bekannten Hybridisierungsvarianten, wie z.B. Festphasen-Hybridisierung, Hybridisierung in Lösung, Sandwich-Hybridisierung, Zwei-Komponenten-Hybridisierung können verwendet werden. Der Nachweis erfolgt in üblicher Weise über eine radioaktive oder nicht-radioaktive Markierung der Sonde.

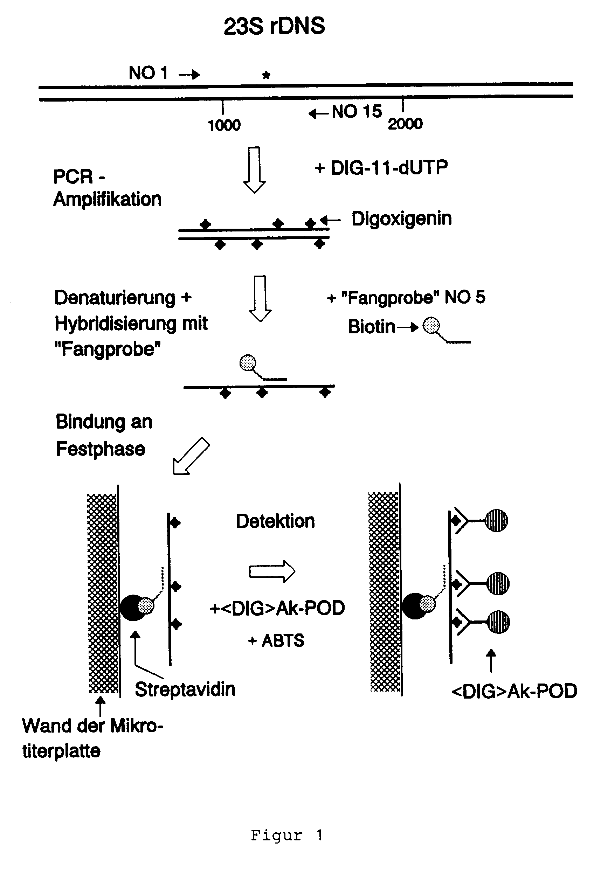

Die Erfindung wird durch die folgenden Beispiele in Verbindung mit den Sequenzprotokollen 1-15, welche die erfindungsgemäßen Nukleinsäure-Sonden zeigen, und Fig. 1, welche einen nichtradioaktiven Nachweis von Neisseria gonorrhoeae zeigt, näher erläutert.

### Beispiel 1

Die Präparation der bakteriellen chromosomalen DNA der unten genannten Spezies erfolgt nach dem von Stern et al. (Cell 37 (1984), 447) beschriebenen Protokoll.

Mit Hilfe einer Slot-Blot-Apparatur wird die chromosomale DNA (jeweils 250 ng) der folgenden Spezies auf einen Nitrocellulosefilter aufgebracht:
- pathogene Neisseria-Spezies:
   Neisseria gonorrhoeae
   Neisseria meningitidis
- apathogene Neisseria-Spezies:
   N.lactamica
   N.mucosa
   N.subflava
   N.sicca
   N.elongata
   N.cinerea
   N.flava
   N.denitrificans

Die Filter werden hierzu mit 2 x Puffer (2x Puffer: 2 mol/l NaCl, 50 mmol/l Tris-HCl, pH 7,5 und 1 mmol/l EDTA) angefeuchtet und eingespannt und ein Vakuum angelegt. Die DNA (Konzentration 0,1 - 1µg/µl) wird mit 50 µl 50 mmol/l Tris-HCl, pH 7,5/5 mmol/l EDTA versetzt und zur Denaturierung 3 Minuten gekocht. Anschließend wird der Ansatz sofort auf Eis überführt, 50 µl 2x Puffer zugegeben und diese Lösung in die Slots der Slot-Blot Apparatur pipettiert. Die Slots werden mit 100 µl 1x Puffer (2x Puffer 1:1 mit Wasser verdünnt) nachgespült, der Filter der Apparatur entnommen, luftgetrocknet und im Vakuum 2 Stunden bei 80°C gebacken.

Die Hybridisierung mit den erfindungsgemäßen Oligonukleotiden erfolgt in üblicher Weise. Die Nitrocellulosefilter werden zunächst zwei Stunden in 1xVHP vorhybridisiert (2xVHP: 0.1% Rinderserumalbumin, 0.1% Ficoll 400.000, 0.1% Polyvinylpropylidon, 1% Glycerin, 1,8 mol/l NaCl, 50 mmol/l Na₂HPO₄, 50 mmol/l NaH₂PO₄, 10 mmol/l EDTA und 10 mg/ml hitzedenaturierte Heringssperma-DNA).

Zur Hybridisierung wird der VHP entfernt und durch die ³²P-markierte Oligonukleotid-Sonde in Hybridisierungspuffer (HP) ersetzt (HP: 1xVHP; Markierung der Probe mit dem Random Primed DNA Labelling kit, Boehringer Mannheim GmbH, Katalog-Nr. 1004 760, gemäß Angaben des Herstellers). Die Hybridisierung erfolgt in einem Hybridisierungsofen bei 40°C. Der HP wird vor Verwendung auf 80°C erhitzt. Nach einer Hybridisierungszeit von mindestens 6 Stunden werden die Filter in Waschpuffer (WP: 0.36 mol/l NaCl, 10 mmol/l Na₂HPO₄, 10 mmol/l NaH₂PO₄, 2 mmol/l EDTA, 0.05 % SDS) zunächst zweimal bei Raumtemperatur und dann zweimal bei 42°C gewaschen. Die Waschtemperatur wird je nach GC-Gehalt der Probe bis auf maximal 60°C erhöht. Die tatsächlichen maximalen Waschtemperaturen der untersuchten Sonden, bei der eine optimale Diskriminierung der Spezies-DNA möglich ist, sind in Tabelle II wiedergegeben.

**Tabelle II**

| Sonde | Waschtemperatur (°C) |
|---|---|
| SEQ ID NO 1 | 52 |
| SEQ ID NO 3 | 52 |
| SEQ ID NO 6 | 48 |
| SEQ ID NO 7 | 48 |
| SEQ ID NO 9 | 58 |
| SEQ ID NO 11 | 48 |
| SEQ ID NO 13 | 48 |

Die Filter werden anschließend in üblicher Weise gegen einen Röntgenfilm exponiert und ausgewertet.

In Tabelle III sind die Ergebnisse der Hybridisierung der verschiedenen Nukleinsäure-Sonden mit pathogenen und apathogenen Spezies gezeigt.

**Tabelle III**

| Spezies* (Stamm/Isolat Nr.) | | Sonde (SEQ ID NO..) | | | | | | |
|---|---|---|---|---|---|---|---|---|
| | | 1 | 3 | 6 | 7 | 9 | 11 | 13 |
| N.gonorrhoeae | MS11 | + | + | + | + | + | + | + |
| N.gonorrhoeae | r2 | + | + | + | + | + | + | + |
| N.gonorrhoeae | R16 | + | + | + | + | + | + | + |
| N.gonorrhoeae | r21 | + | + | + | + | + | + | + |
| N.gonorrhoeae | 510 | + | + | + | + | + | + | + |
| N.gonorrhoeae | 514 | + | + | + | + | + | + | + |
| N.meningitidis | B | - | - | - | - | - | - | - |
| N.meningitidis | C | - | - | - | - | - | - | - |
| N.meningitidis | D | - | - | - | - | - | - | - |
| N.meningitidis | 2-4 | - | - | - | - | - | - | - |
| N.meningitidis | 3-1 | - | - | - | - | - | - | - |
| N.lactamica | 1855 | - | - | - | - | - | - | - |
| N.lactamica | 3272 | - | - | - | - | - | - | - |
| N.mucosa | 112 | - | - | - | - | - | - | - |
| N.mucosa | 114 | - | - | - | - | - | - | - |
| N.subflava | 124 | - | - | - | - | - | - | - |
| N.sicca | 118 | - | - | - | - | - | - | - |
| N.sicca | 2844 | - | - | - | - | - | - | - |
| N.elongata | 129 | - | - | - | - | - | - | - |
| N.cinerea | 126 | - | - | - | - | - | - | - |
| N.cinerea | 2199 | - | - | - | - | - | - | - |
| N.flava | 122 | - | - | - | - | - | - | - |
| N.flava | 123 | - | - | - | - | - | - | - |
| N.denitrificans | 2950 | - | - | - | - | - | - | - |

| | | | | | | | | |
|---|---|---|---|---|---|---|---|---|
| * klassifiziert nach Bergey's Manual of Systematic Bacteriology, Krieg N.R., Holt G., eds., Williams and Wilkins, Baltimore (1984), 288-298 | | | | | | | | |

Zur Überprüfung der Spezifität der Nukleinsäure-Sonden wird in einem zweiten Hybridisierungsansatz die genomische DNA von nicht-Neisseria-Spezies (u.a.auch Besiedler der menschlichen Schleimhaut) als weitere Kontrolle miteinbezogen.

Das Ergebnis dieser Hybridisierung ist in Tabelle IV aufgeführt.

**Tabelle IV**

| Spezies (Stamm/Isolat Nr.) | | Sonde (SEQ ID NO ...) | | | | | | |
|---|---|---|---|---|---|---|---|---|
| | | 1 | 3 | 6 | 7 | 9 | 11 | 13 |
| N.gonorrhoeae | R16 | + | + | + | + | + | + | + |
| N.gonorrhoeae | 514 | + | + | + | + | + | + | + |
| N.lactamica | 3272 | - | - | - | - | - | - | - |
| N.elongata | 129 | - | - | - | - | - | - | - |
| N.sicca | 2844 | - | - | - | - | - | - | - |
| N.cinerea | 2199 | - | - | - | - | - | - | - |
| N.denitrificans | 2950 | - | - | - | - | - | - | - |
| Moraxella bovis | D1931 | - | - | - | - | - | - | - |
| E.coli | GM 48 | - | - | - | - | - | - | - |
| Haemophilus influenzae | 2214 | - | - | - | - | - | - | - |
| Haemophilus parainfluenzae | 1207 | - | - | - | - | - | - | - |
| Proteus vulgaris | 770 | - | - | - | - | - | - | - |
| Pseudomonas putida | 574 | - | - | - | - | - | - | - |
| Bacillus alvei | 3006 | - | - | - | - | - | - | - |
| Leuconostoc mesenteroides | 83 | - | - | - | - | - | - | - |
| Listeria monocytogenes | 1/2a | - | - | - | - | - | - | - |
| Staphylococcus aureus | D1901 | - | - | - | - | - | - | - |
| Staphylococcus epidermidis | 1050 | - | - | - | - | - | - | - |
| Streptococcus pneumoniae | 3028 | - | - | - | - | - | - | - |
| Steptomyces griseus | A | - | - | - | - | - | - | - |
| Isolat aus Mundabstrich | | - | - | - | - | - | - | - |

### Beispiel 2

### Nicht-radioaktiver Nachweis von Neisseria gonorrhoeae

Für einen nicht-radioaktiven Nachweis von Neisseria gonorrhoeae wird zunächst ein spezifisches DNA-Fragment über eine Polymerase-Kettenreaktion amplifiziert, wobei die Fragmente über den Einbau von DIG-11-dUTP gleichzeitig markiert werden. Diese markierten Fragmente werden dann zu Oligonukleotiden gegeben, die mit diesen Fragmenten hybridisieren können und somit als Fangprobe fungieren. Die Immobilisierung erfolgt dann über eine Kopplung dieser Oligonukleotide am 3'-Ende mit Biotin, über das diese Oligonukleotide an eine mit Streptavidin beschichtete Mikrotiterplatte gebunden werden. Unspezifische PCR-Fragmente, die nicht mit dem als Fangprobe fungierenden Oligonukleotid hybridisieren können, werden bei anschließenden Waschschritten entfernt. Der Nachweis der gebundenen spezifischen DNA-Fragmente erfolgt über der Digoxigenin-Markierung nach Zugabe eines POD-konjugierten Antikörpers gegen Digoxigenin und ABTS®-Substratlösung für die Peroxidasereaktion. Eine Bindung des amplifizierten DNA-Fragments an die Fanggruppe ist dann über die Umsetzung des Substrats, die im Photometer gemessen werden kann, erkennbar (s. Fig. 1).

Zur Versuchsdurchführung werden zunächst die Vertiefungen einer 96er-Mikrotiter-Platte gemäß EP-A 0 344 578 mit Streptavidin (100 µl einer Lösung von 1 µg/ml in PBS) bei 4°C über Nacht beschichtet und noch freie unspezifische Bindungsstellen durch Inkubation mit 300 µl BSA (Rinderserumalbumin 10 mg/ml) für 2 h bei Raumtemperatur blockiert.

Als PCR-Primer für die Amplifikation eines spezifischen DNA-Fragmentes aus chromosomaler Gonokokken-DNA werden die Oligonukleotide SEQ ID NO 1 und 15 ausgewählt. Die Hybridisierung dieser Primer erfolgt bei einer Temperatur von 57°C. Die weitere PCR erfolgt in bekannter Weise. Das so erhaltene Produkt hat eine Länge von 750 bp. 20 µl des Amplifikationsansatzes werden zur Denaturierung der DNA-Fragmente mit 20 µl TE-Puffer und 10 µl 0,5 mol/l NaOH versetzt und 10 min bei Raumtemperatur inkubiert. Dieser Denaturierungsansatz wird durch Zugabe von 450 µl angesäuerter Hybridisierungslösung (50 mmol/l Na-Phosphatpuffer, pH 6,8, 0,05 mol/l HCl, 0,75 mol/l NaCl, 75 mmol/l Na-Citrat, 0,05 % RSA, pH 5,4) neutralisiert. Dieser Lösung wird das Biotin-markierte Oligonukleotid ("Fangprobe", SEQ ID NO 5) beigefügt (100 ng/ml). 200 µl dieses Hybridisierungsansatzes werden in Streptavidin-beschichtete Mikrotiterplatten gegeben und 3 h bei 37°C inkubiert. Über das Biotin-markierte Oligonukleotid wird das DNA-Fragment an die Streptavidin-Beschichtung der Mikrotiterplatte gebunden. Die Platten werden anschließend dreimal mit 0,9 % NaCl-Lösung gewaschen, dann werden 200 µl Konjugatlösung (200 mU/ml 〈DIG〉-PolyPOD-Konjugat (Fab:POD = 1:1) in 100 mmol/l Tris/HCl, pH 7,5, 0,9 % NaCl, 1 % RSA) zugegeben und es wird 30 min bei 37°C inkubiert. Nach dreimaligem Waschen mit 0,9 % NaCl-Lösung wird die Substratumsetzung durch Zugabe von 200 µl ABTS®-Substratlösung (2,2'-Azino-di-[3-ethyl-benzthiazolin-sulfonat] (1,9 mmol/l)) (ABTS®: Boehringer Mannheim GmbH, Katalog-Nr. 756407 in ABTS®-Puffer: Boehringer Mannheim GmbH, Katalog-Nr. 1204530) gestartet. Nach 5-15 min Inkubation bei 37°C wird der Farbumschlag im Photometer (Easy Reader EAR 400 FW Mikrotiterplatten Reader, SLT-Labinstruments, Groedig/Salzburg) bei 405 nm gemessen.

## Patentansprüche

1. Neisseria gonorrhoeae-spezifische Nukleinsäuresonde dadurch gekennzeichnet, daß sie mindestens eine der in SEQ ID NO 1-15 gezeigten Sequenzen enthält.

2. Sonde nach Anspruch 1 dadurch gekennzeichnet, daß sie an ihrem 5'- und/oder 3'-Ende weitere Nukleotide aufweist.

3. Sonde nach Anspruch 2 dadurch gekennzeichnet, daß sie an ihrem 5'- und/oder 3'-Ende bis zu 8 weitere Nukleotide aufweist.

4. Sonde nach Anspruch 2 oder 3 dadurch gekennzeichnet, daß die weiteren Nukleotide denjenigen Nukleotiden entsprechen, die an dieser Stelle im natürlichen 23S rRNA-Gen, von dem sich diese Sonden ableiten, vorhanden sind.

5. Sonde nach einem der Ansprüche 1-4 dadurch gekennzeichnet, daß sie aus modifizierten oder nicht-modifizierten Ribonukleotiden oder Desoxyribonukleotiden besteht.

6. Sonde nach einem der Ansprüche 1-5 dadurch gekennzeichnet, daß sie in einer oder mehreren Kopien auf einem einzel- oder doppelsträngigen Vektor enthalten ist.

7. Sonde nach einem der Ansprüche 1-6 dadurch gekennzeichnet, daß sie in markierter Form vorliegt.

8. Verfahren zum Nachweis der pathogenen Neisseria-Spezies N.gonorrhoeae durch Hybridisierung von DNA oder RNA der zu untersuchenden Probe mit einer Sonde und Nachweis der Hybridbildung, dadurch gekennzeichnet, daß man eine Sonde nach einem der Ansprüche 1-7 verwendet.

9. Verfahren nach Anspruch 8 dadurch gekennzeichnet, daß man die Hybridbildung über die Markierung der Sonden nachweist.

## Claims

1. Neisseria gonorrhoeae-specific nucleic acid probe, wherein it contains at least one of the sequences shown in SEQ ID NO 1-15.

2. Probe as claimed in claim 1, wherein it has further nucleotides at its 5' and/or 3' end.

3. Probe as claimed in claim 2, wherein it has up to 8 further nucleotides at its 5' and/or 3' end.

4. Probe as claimed in claim 2 or 3, wherein the further nucleotides correspond to those nucleotides which are present at this site in the natural 23S rRNA gene from which these probes are derived.

5. Probe as claimed in one of the claims 1-4, wherein it is composed of modified or non-modified ribonucleotides or deoxyribonucleotides.

6. Probe as claimed in one of the claims 1-5, wherein it is present in one or several copies on a single-stranded or double-stranded vector.

7. Probe as claimed in one of the claims 1-6, wherein it is present in a labelled form.

8. Method for the detection of the pathogenic Neisseria species N.gonorrhoeae by hybridizing DNA or RNA of the sample to be examined with a probe and detecting hybrid formation, wherein a probe as claimed in one of the claims 1-7 is used.

9. Method as claimed in claim 8, wherein the hybrid formation is detected by means of the labelling of the probes.

## Revendications

1. Sonde d'acide nucléique spécifique à Neisseria gonorrhoeae, caractérisée en ce qu'elle contient au moins une des séquences montrées dans les SEQ ID NO 1 - 15.

2. Sonde selon la revendication 1, caractérisée en ce qu'elle présente des nucléotides supplémentaires à son extrémité 5' et/ou 3'.

3. Sonde selon la revendication 2, caractérisée en ce qu'elle présente jusqu'à 8 nucléotides supplémentaires à son extrémité 5' et/ou 3'.

4. Sonde selon la revendication 2 ou 3, caractérisée en ce que les nucléotides supplémentaires correspondent aux nucléotides qui sont présents à cet endroit dans le gène naturel de l'ARNr 23S, duquel sont dérivées ces sondes.

5. Sonde selon l'une des revendications 1 à 4, caractérisée en ce qu'elle est constituée de ribonucléotides ou de désoxyribonucléotides modifiés ou non-modifiés.

6. Sonde selon l'une des revendications 1 à 5, caractérisée en ce qu'elle est contenue en une ou plusieurs copies dans le vecteur simple brin ou double brin.

7. Sonde selon l'une des revendications 1 à 6, caractérisée en ce qu'elle se présente sous forme marquée.

8. Procédé de détection de l'espèce pathogène de Neisseria, N. gonorrhoeae, par hybridation d'ADN ou d'ARN de l'échantillon à analyser au moyen d'une sonde et par détection de la formation d'hybrides, caractérisé en ce qu'on utilise une sonde selon l'une des revendications 1 à 7.

9. Procédé selon la revendication 8, caractérisé en ce qu'on détecte la formation d'hybrides par le marquage des sondes.
